# EUROPÄISCHE PATENTSCHRIFT

(11) **EP 1 989 293 B1**
(45) Veröffentlichungstag und Bekanntmachung des Hinweises auf die Patenterteilung: **05.01.2011**
(21) Anmeldenummer: 07721969.9
(22) Anmeldetag: 28.02.2007
(51) Int. Cl.: A61L 27/38

(54) **PRÄVASKULARISIERTE GEWEBETRANSPLANTATKONSTRUKTE ZUR REKONSTRUKTION EINES MENSCHLICHEN ODER TIERISCHEN ORGANS**
PREVASCULARIZED TISSUE TRANSPLANT CONSTRUCTS FOR THE RECONSTRUCTION OF A HUMAN OR ANIMAL ORGAN
CONSTRUCTIONS DE TRANSPLANTATION TISSULAIRE PRÉVASCULARISÉES DESTINÉES À LA RECONSTRUCTION D'UN ORGANE HUMAIN OU ANIMAL

(30) Priorität: 28.02.2006 DE 102006009539
(43) Veröffentlichungstag der Anmeldung: 12.11.2008
(73) Patentinhaber: UroTec GmbH, 01069 Dresden (DE)
(72) Erfinder: RAM-LIEBIG, Gouya, 01309 Dresden (DE); WIRTH, Manfred, 01326 Dresden (DE)
(74) Vertreter: Carlsohn, Alexander
(86) Internationale Anmeldenummer: PCT/DE2007/000368
(87) Internationale Veröffentlichungsnummer: WO 2007/098742

(56) Entgegenhaltungen:
- WO-A-2006/060779
- DE-B3-102004 037 184
- US-A1- 2001 051 824
- US-A1- 2004 006 395
- SCHULTHEISS ET AL: "BIOLOGICAL VASCULARIZED MATRIX FOR BLADDER TISSUE ENGINEERING: MATRIX PREPARATION, RESEEDING TECHNIQUE AND SHORT-TERM IMPLANTATION IN A PORCINE MODEL" JOURNAL OF UROLOGY, BALTIMORE, MD, US, Bd. 173, Nr. 1, Januar 2005 (2005-01), Seiten 276-280, XP005529573 ISSN: 0022-5347 in der Anmeldung erwähnt
- DATABASE BIOSIS [Online] BIOSCIENCES INFORMATION SERVICE, PHILADELPHIA, PA, US; August 2002 (2002-08), VELAZQUEZ OMAIDA C ET AL: "Fibroblast-dependent differentiation of human microvascular endothelial cells into capillary-like 3-dimensional networks" XP002444607 Database accession no. PREV200200441299 & FASEB JOURNAL, Bd. 16, Nr. 10, August 2002 (2002-08), Seiten 1316-1318, ISSN: 0892-6638
- M. MARKOWICZ ET AL: "Topics in Tissue Engineering, volume 2 - VI Angiogenesis & Vascularisation - Chapter 6: Enhancing the Vascularization of Three-Dimensional Scaffolds: New Strategies in Tissue Regeneration and Tissue Engineering" [Online] 2005, N. ASHAMMAKHI & R.L. REIS , XP002444573 Gefunden im Internet: URL:http://www.oulu.fi/spareparts/ebook_to pics_in_t_e_vol2/abstracts/markowicz1_0102 .pdf> [gefunden am 2007-07-26] das ganze Dokument

## Beschreibung

Die Erfindung betrifft Gewebetransplantatkonstrukte zur Rekonstruktion eines menschlichen oder tierischen Organs, ein Verfahren zur Herstellung eines solchen Gewebetransplantatkonstruktes sowie Verwendungen des Gewebetransplantatkonstruktes. Die Erfindung betrifft insbesondere ein Gewebetransplantatkonstrukt zur Rekonstruktion der unteren urologischen Organe und insbesondere der Harnblase.

Das Ziel der Gewebearchitektur ("tissue engineering") ist der Ersatz von geschädigtem, verletztem oder fehlendem Körpergewebe durch biologisch verträgliches Ersatzmaterial. Derzeit werden zur Beschleunigung der Geweberegeneration sowohl die zellbeschichtete Technik als auch die nicht-zellbeschichtete Technik in der Gewebearchitektur erforscht (Alberti et al., 2004). Die zellbeschichtete Technik (oder zelluläre Gewebearchitektur) nutzt biologisch abbaubare Membranen, die *in vitro* mit primär kultivierten Zellen beschichtet wurden, wobei die Zellen aus einer Biopsie von nativem Wirtsgewebe erhalten worden sind. Dieses zusammengesetzte Transplantat wird dann in den Wirt zur Fortsetzung des regenerativen Prozesses gebracht. Die nicht-zellbeschichtete Technik umfaßt die direkte *in vivo*-Plazierung eines unbeschichteten biologisch abbaubaren Materials in den Wirt, das dann als Gerüst fungieren soll, damit der natürliche Regenerierungsprozeß *in vivo* stattfinden kann. Diese Techniken unterstützen eine Geweberegenerierung, die der normalen Embryonalentwicklung des interessierenden Organs ähnelt.

Azelluläre biologische Materialien zeigen gute Ergebnisse bei der Gewebegeneration *in vivo* als zellbeschichtete und nicht-zellbeschichtete Membranen (Atala et al., 2000). Tatsächlich sind diese biologisch abbaubaren Materialien in der Gewebearchitektur von unterschiedlichen Organen als ein natürliches, abbaubares und poröses Material verwendet worden, und sind dafür bekannt, biologisch spezifische Signale zur molekularen Interaktion mit den kultivierten Zellen *in vitro* bereitzustellen und außerdem mit den Zellen des Zielgewebes nach der Implantation zu interagieren.

Wenn jedoch das Transplantat einmal in den Wirt implantiert ist, ist die Fähigkeit, die Zellen am Leben zu halten, die auf der Oberfläche oder innerhalb der Matrix *in vitro* wuchsen (zellbeschichtete Technik) oder die die Matrix nach der Implantation infiltrieren (nicht-zellbeschichtete Technik), ein kritisches Hindernis. Es ist gezeigt worden, daß ein Stück des Gewebes mit einem Volumen, das wenige Kubikmillimeter übersteigt, nicht durch Diffusion von Nährstoffen überleben kann, sondern die Anwesenheit von Blutkapillaren für die Zufuhr von wichtigen Nährstoffen und Sauerstoff erfordert (Mooney et al.,1999). Daher kann die verzögerte Vaskularisation ein Implantatversagen verursachen. Bis heute beschränkten sich die Erfolge der Implantationstechniken auf relativ dünne oder avaskuläre Strukturen (beispielsweise Haut und Knorpel), wo die Gefäßbildung nach der Implantation durch den Wirt ausreichend ist, um die Impläntatanforderungen an Sauerstoff und. Nährstoffen zu erfüllen (Jam et al., 2005). Die Vaskularisation bleibt ein kritisches Hindernis bei der Entwicklung von dickeren, metabolisch anspruchsvollen Organen, wie Herz, Hirn und Harnblase.

Die Fähigkeit zur Prävaskularisierung von Gewebegerüsten ist eine prinzipielle therapeutische Strategie und ein signifikanter Schritt in der Gewebetechnik durch die Vermeidung eingeschränkter Geweberegeneration. Eine prävaskularisierte Matrix beschleunigt ihre Vaskularisation und verbessert ihre Durchblutung und ihr Überleben *in vivo.* Besonders komplexe Gewebe und Organe benötigen eine vaskuläre Versorgung, um das Überleben des Transplantats zu garantieren und um bioartifizielle Organe funktionsfähig zu machen (Mertsching et al., 2005). Eine Methode der Neovaskularisation ist die Bildung von neuen Gefäßen aus Endothelzellen. Dieses Verfahren, das als Vaskulogenese (Gefäßbildung) bezeichnet wird, findet normalerweise während der Embryonalentwicklung beim Ausbilden von Organen statt (Risau et al., 1995).

Von besonderer Bedeutung ist die Prävaskularisation von Materialien zur Rekonstruktion der unteren Harnwege (Harnblase, Harnleiter und Harnröhre). Diese Organe weisen eine reiche Versorgung über Blutgefäße auf. In der zellbeschichteten Technik der Gewebearchitektur der unteren Harnorgane beschränkten sich die Versuche bis jetzt lediglich auf die Kultivierung von Urothelzellen und Muskelzellen (Alberti et al., 2004) auf biologischen Membranen. Diese Zellen können leicht aus kleinen Blasenbiopsien geerntet werden.

Die offene Frage ist gegenwärtig, welche Art von Endothelzellen zur Prävaskularisation von biologischen Gerüsten, die *in vivo* implantiert werden sollen, verwendet werden können. Es ist bekannt, daß die Phänotypen von Endothelzellen in Abhängigkeit des Gefäßtyps und des Organs stark variieren, und diese Gewebespezifität eine wichtige Rolle in der lokalen Physiologie der Organe spielt (Jam et al., 2005).

Schultheiß et al. beschreiben in "Biological vascularized matrix for bladder tissue engineering: matrix preparation, reseeding technique and short-term implantation in a porcine model", J. Urol. Jan 2005; 173 (1): 276-80, eine biologische azelluläre Matrix, die mit Glattmuskelzellen, Urothelzellen und endothelialen Vorläuferzellen besiedelt ist. Die Vorläuferzellen wurden aus Blutzellfraktionen gewonnen. Zur Herstellung eines Gewebetransplantatkonstruktes wird die Matrix zusätzlich zu mit Glattmuskelzellen und Urothelzellen mit endothelialen Vorläuferzellen aus dem Blut beschichtet. Diese Zellen sind aber nicht in der Lage, vaskuläre Strukturen in der Matrix zu bilden. Zusätzlich sind die endotheliale Vorläuferzellen nicht organspezifisch. Sie sind außerdem keine reife Endothelzellen.

Aufgabe der Erfindung ist es, die Nachteile nach dem Stand der Technik zu beseitigen. Es soll insbesondere ein Gewebetransplantatkonstrukt angegeben werden, daß im Falle einer Beschichtung der Membran mit Endothelzellen (und die Bildung von vaskulären strukturen innerhalb der Membran) eine schnelle und bessere Generierung des umgrenzenden Gewebes in dem Gerüst nach dem Implantation oder daß im Falle einer Beschichtung der Membran mit Endothelzellen sowie weiteren organspezifischen Zellen (wie Urothelzellen, Muskelzellen und/oder interstitiellen Zellen) eine bessere Versorgung der in ihm enthaltenen Zellen nach der Implantation ermöglicht.

Diese Aufgabe wird durch die Merkmal der Ansprüche 1, und 7, gelost. Zweckmäßige Ausgestaltungen der Erfindung ergeben sich aus den Merkmalen der Unteransprüche.

US 2004/0006395 A1 beschreibt eine Membran, die ausschließlich mit Endothelzellen besiedelt ist, in der aber keine mikrovaskulären Strukturen ausgebildet sind, weil die Endothelzellen nicht in die Membran eindringen; oder (ii) eine Membran, die mit Endothelzellen und weiteren Zellen (Glattmuskelzellen oder 3T3-Zellen) besiedelt ist und in der mikrovaskuläre Strukturen ausgebildet sind.

Velazquez Omaida C. et al. (Database Biosis [Online] Biosciences Information Service, Philadelphia, PA, US, August 2002 (2002-08) "Fibroblast-dependent differentiation of human microvascular endothelial cells into capillary-like 3-dimensiorial networks" (Datenbank-Zugangs-Nr. PREV200200441299) offenbaren, daß Endothelzellen mit humanem Kollagen I und mit einer zweiten Schicht aus Kollagen mit eingebetteten Fibroblasten zur Ausbildung von dreidimensionalen kapillarähnlichen Netzwerken überschichtet werden.

Hopkins Richard et al. (www.oulo.fi) offenbaren eine Methode zur Beschleunigung der Proliferation von HUVEC-Zellen auf vernetztem Kollagen mit Hilfe von mesenchymalen Knochenmarkstammzellen und dermalen Fibroblasten.

US 2001/051824 A1 lehrt eine Membran, die mit Myofibroblasten besiedelt worden ist. Die Kultivierung der Myofibroblasten erfolgt unter pulsierenden Flußbedingungen. Die Membran kann zusätzlich auch mit Endothelzellen besiedelt werden.

DE 10 2004 037 184 B3 offenbart ein Verfahren zur Herstellung eines Gewebetransplantatkonstruktes, daß (a) das Aufbringen von organ-spezifischen Zellen (konkret offenbart sind im dortigen Beispiel Urothelzellen) auf eine biologische Membran, (b) die Proliferation dieser Zellen unter stromaler Induktion und (c) die terminale Differenzierung eines Teiles der expandierten Urothelzellen umfaßt, wodurch eine Membran erhalten wird, die mehrere Schichten von Urothelzellen aufweis. Nach Maßgabe der Erfindung ist ein Gewebetransplantatkonstrukt zur Rekonstruktion eines menschlichen oder tierischen Organs vorgesehen, bestehend aus
(a) einer biologisch kompatiblen azellulären Membran; und
(b) mikrovaskulären Endothelzellen, die die Membran durchdringen, wobei die mikrovaskulären Endothelzellen dermale mikrovaskuläre Endothelzellen oder mikrovaskuläre BlasenEndothelzellen sind;
wobei innerhalb der Membran mikrovaskuläre Strukturen aus den mikrovaskulären Endothelzellen ausgebildet sind.

Die mikrovaskulären Strukturen, die aus den aufgebrachten mikrovaskulären Endothelzellen gebildet sind, ermöglichen eine Versorgung der übrigen. Zellen der unteren Harnorgange, insbesondere Urothelzellen, Muskelzellen, interstitielle Zellen, nach der Implantation des Konstruktes mit Nährstoffen und Sauerstoff. Ebenso werden auch andere Zellen versorgt, die nach der Implantation in das Konstrukt eindringen.

Das erfindungsgemäße Gewebetransplantatkonstrukt kann weitere, gewebespezifische Zellen, beilspielsweise Urothelzellen und/oder Glattmüskelzellen und/oder interstitielle Zellen umfassen, die vor der Implantation auf die Membran aufgebracht und dort kultiviert worden sind. Auch diese Zellen werden dann nach der Implantation des Gewebetransplantatkonstruktes von der mikrovaskulären Struktur versorgt.

Die Erfindung stellt somit erstmals ein Gewebetransplantatkonstrukt zu Verfügung, daß eine Versorgung von Zellen innerhalb des Konstruktes und nicht nur an dessen Oberfläche ermöglicht. Die Konstrukte sind daher hervorragend zur Rekonstruktion von Organen geeignet.

Die Erfindung verwendet insbesondere organ-spezifische mikrovaskulären Endothelzellen der Harnblase (mikrovaskuläre Blasen-Endothelzellen) zur Prävaskularisation von Matrizes, die zur Rekonstruktion der unteren Harnorgane verwendet werden sollen. Die mikrovaskulären Blasen-Endothelzellen konnten aus einem kleinen Biopsiematerial aus diesem Organ isoliert werden und verwendet werden, um Endothelgefäßnetzwerke in unterschiedlichen biologischen azellulären Matrizes zu bilden. Bevorzugte Matrizes sind azelluläre Blasen-Matrix, azelluläre Harnröhrenmatrix, azelluläre Blasensubmukosa, Dünndarmsubmukosa, azelluläre Hautmatrix, azelluläre Aortamatrix.

Alternativ zu mikrovaskulären Blasen-Endothelzellen können auch dermale mikrovaskuläre Endothelzellen verwendet werden. Dermalen mikrovaskuläre Endothelzellen können auch zur Herstellung eines. Gewebetransplantatkonstruktes verwendet werden, daß zur Rekonstruktion der unteren Harnorgane, aber auch anderer Organe benutzt werden kann. Zur Rekonstruktion der Harnblase wird jedoch die Verwendung von mikrovaskulären Blasen-Endothelzellen aufgrund der Gewebespezifität bevorzugt.

Die Erfindung ermöglicht insbesondere die *in vitro-*Kultivierung von mikrovaskulären Endothelzellen auf unterschiedlichen biologischen Matrizes und die Bildung einer stabilen vaskuläre Netzwerkkonstruktion für die Rekonstruktion von Weichteilgewebe insbesondere der Harnblase, Harnleiter und Harnröhre. Zur Kultivierung erfolgt in Kultursystemen, die angiogene Wachstumsfaktoren bereitstellten, um die Prävaskularisation von Matrizes mit mikrovaskulären Blasen-Endothelzeilen zu induzieren. Die Bildung von Gefäßstrukturen in den fertigen Gewebetransplantatkonstrukturen wird durch Blasenstromazellen, Knochenmarksstroma-Progenitorzellen und Blasenurothelzellen, insbesondere durch ein Medium, das mit diesen Zellen konditioniert worden ist, induziert. Die Stromazellen, Knochenmarksstroma-Progenitorzellen und Epithelzellen wie Urothelzellen sind bekannt dafür, das sie mit der Induktion der Blutgefäßbildung *in vitro* in. Verbindung stehen (Velazquez et al., 2002; Markowicz et al., 2005; Thompson et al., 2006).

Die Erfindung ist jedoch nicht auf ein Gewebetransplantatkonstrukt zur Rekonstruktion der unteren Harnleiter beschränkt, sondern kann auch auf andere Organe, insbesondere Weichteilorgane übertragen werden.

Der Begriff "organ-spezifisch" soll hierbei so verstanden werden, daß Zellen desselben oder eines gleichen Organs, das rekonstruiert werden soll, auf die Membran aufgebracht werden. Soll beispielsweise eine Harnblase rekonstruiert werden, so sind hier unter organ-spezifischen mikrovaskulären Endothelzellen mikrovaskulären Endothelzellen aus der Harnblase zu verstehen.

Organe sind funktionelle Einheiten des Körpers. Bevorzugtes Beispiel ist die Harnblase.

Unter dem Begriff "mikrovaskuläre Struktur" ist die Ausbildung einer kapillaren Struktur innerhalb der Membran zu verstehen. Eine kapillare Struktur besteht vorzugsweise aus stehen. Eine kapillare Struktur besteht vorzugsweise aus strangförmigen und/oder röhrchenförmigen Einheiten, die aus mikrovaskulären Endothelzellen gebildet sind. Die röhrchenförmigen Einheiten weisen vorzugsweise vollständig ausgebildeten Lumina auf. Die strangförmigen und/oder röhrchenförmigen Einheiten sind vorzugsweise miteinander vernetzt. Nach der Implantation des erfindungsgemäßen Konstruktes in das zu rekonstruierende Organ und der Verbindung der mikrovaskulären Gefäßstruktur des Konstruktes mit der *in vivo*-Gefäßstruktur wird die Versorgung der Endothelzellen und anderen Zellen des Konstruktes sicherstellt. Das Konstrukt kann somit über die mikrovaskulären Strukturen unmittelbar nach der Implantation mit Nährstoffen und Sauerstoff versorgt werden. Der Prozeß der Blutgefäßbildung wird auch als Vaskularisation bezeichnet. Unter Prävaskularisation wird verstanden, daß die mikrovaskuläre Struktur schon vor der Implantation vorhanden ist.

Die Begriffe "Membran" und "Matrix" werden hier, soweit nicht anders angegeben, synonym verwendet. Die Membran sollte die Komponenten der extrazellulären. Matrix (ECM), insbesondere deren Wachstumsfaktoren, enthalten. Vorzugsweise ist die Membran eine biologische Membran. Die Membran bildet das dreidimensionale biologische Gerüst, in das die aufgebrachten mikrovaskulären Endothelzellen während der Kultivierung eindringen.

Bei der Membran handelt es sich vorzugsweise um eine azelluläre humane oder porzine Harnblase, azelluläre humane oder porzine Harnblasensubmukosa, azelluläre humane oder porzine Dermis, azellulären humanen oder porzinen Dünndarm, azellulären humane oder porzine Aorta oder azelluläre humanen oder porzine Harnröhre. Die Membran ist kollagenhaltig. Zweckmäßigerweise ist die Membran mechanisch stabil.

Die äußere Oberfläche der Membran ist vorzugsweise bis zu 40 cm² groß, kann aber auch größer sein. Die Dicke der Membran liegt vorzugsweise zwischen 100 µm und 100 mm.

Unter Rekonstruktion wird der Ersatz von beschädigten oder kranken Bereichen eines menschlichen oder tierischen Organs, insbesondere einer Harnblase, einer Harnröhre oder eines Harnleiters verstanden.

Der Begriff "Durchdringen" soll hier so verstanden werden, daß mikrovaskuläre Endothelzellen in von der Oberfläche der Membran in deren Inneres eingedrungen sind und das Innere der Membran von den mikrovaskulären Endothelzellen besiedelt ist. Der Begriff "Durchdringen" soll hier dem Begriff "Invasion" entsprechen.

Unter stromaler Induktion wird die durch Stroma induzierte Proliferation der mikrovaskulären Endothelzellen verstanden. Vorzugsweise ist die stromale Induktion eine Induktion mittels Blasenstromazellen und Knochenmarksstroma-Progenitorzellen.

Unter epithelialer Induktion wird die durch Epithelzellen induzierte Proliferation der mikrovaskulären Endothelzellen verstanden. Im Falle der unteren Harnwege sind die Epithelzellen Urothelzellen, so daß dann von urothelialer Induktion gesprochen wird.

Unter epithelial-stromaler Induktion wird die durch Proliferation der mikrovaskulären Endothelzellen verstanden, die durch ein Gemisch aus Stromazellen und Epithelzellen induziert wird. Vorzugsweise ist die urthelial-stromale Induktion eine Induktion mittels Urothelial-Blasenstromazellen. Im Falle der unteren Harnwege sind die Epithelzellen Urothelzellen, so daß dann von urothelial-stromaler Induktion gesprochen wird.

Ein Gemisch aus Blasen-Stromazellen und Blasen-Urothelzellen wird nachstehend auch als Urothel-Blasenstromzellen, UrothelStromazellen, Stroma-Urothelzellen oder Blasenurothel-Stromazellen bezeichnet.

Eine Harnblase besteht aus Mukosa (Urothelzellen) und Stroma (Fibroblasten, Glattmusklezellen und Endothelzellen).

Das erfindungsgemäße Gewebetransplantatkonstrukt wird vorzugsweise zur Rekonstruktion der unteren Harnwege, besonders bevorzugt der Harnblase, der Harnröhre oder des Harnleiters verwendet. In diesem Fall ist das menschliche oder tierische Organ, das zu rekonstruieren ist, eine Harnblase ist, während die organ-spezifischen mikrovaskulären Endothelzellen mikrovaskuläre Blasen-Endothelzellen und/oder dermale mikrovaskuläre Endothelzellen sind.

Besonders bevorzugt sind die mikrovaskulären Endothelzellen autologe mikrovaskuläre Endothelzellen, d. h. die mikrovaskulären Endothelzellen werden aus dem Gewebe eines Patienten isoliert, dessen Organ mit dem erfindungsgemäßen Gewebetransplantatkonstrukt rekonstruiert werden soll.

Ebenso bevorzugt können mikrovaskuläre Endothelzellen aus der Haut isoliert werden und zur PrävasKularisierung der Membran verwendet werden.

Nach Maßgabe der Erfindung ist ferner ein Verfahren zur Herstellung des Gewebetransplantatkonstruktes zur Rekonstruktion eines menschlichen oder tierischen Organs vorgesehen, wobei das Gewebetransplantatkonstrukt aus einer Membran und mikrovaskulären Endothelzellen besteht, wobei das Verfahren die Schritte
(a) Isolieren von dermalen mikrovaskulären Endothelzellen oder mikrovaskulären Blasen-Endothelzellen;
(b) Aufbringen der mikrovaskulären Endothelzellen auf eine biologisch kompatible azelluläre Membran;
(c) Kultivieren der mikrovaskulären Endothelzellen, die auf die biologisch kompatible azelluläre Membran aufgebracht worden sind, unter stromaler Induktion oder unter epithelial-stromaler Induktion, unter Ausbildung mikrovaskulärer Strukturen, bestehend aus mikrovaskulären Endothelzellen, in der Membran umfaßt.

Die stromale Induktion wird vorzugsweise unter Verwendung von humanen oder tierischen Blasenstromazellen oder von humanen oder tierischen Knochenmarksstroma-Progenitorzellen durchgeführt.

Das Kultivieren unter stromaler Induktion wird besonders bevorzugt mittels eines konditionierten Mediums durchgeführt, wobei das konditionierte Medium mittels humaner oder tierischer Blasenstromazellen oder humaner oder tierischer Knochenmarksstroma-Progenitorzellen konditioniert worden ist. Ein konditioniertes Medium kann erhalten werden, indem ein nicht-konditioniertes Medium mit humanen oder tierischen Blasenstromazellen oder humanen oder tierischen Knochenmarksstroma-Progenitorzellen kultiviert und das Medium anschließend als Überstand aus den Blasenstromazellen oder Knochenmarksstroma-Progenitorzellen entfernt wird. Das konditionierte Medium wird im folgenden auch als stroma-konditioniertes Medium oder Stromazellen-konditioniertes Medium bezeichnet.

Die epithelial-stromale Induktion ist vorzugsweise eine urothelial-stromale Induktion, die unter Verwendung von Urothel-Blasenstromazellen durchgeführt wird.

Das Kultivieren unter epithelial-stromaler Induktion wird besonders bevorzugt mittels eins konditionierten Mediums durchgeführt, wobei das konditionierte Medium mittels humaner oder tierischer Epithelzellen, insbesondere Urothelzellen, und Blasenstromazellen konditioniert worden ist. Ein konditionierte Medium kann erhalten werden, indem ein nicht-konditioniertes Medium mit humanen oder tierischen Epithelzellen kultiviert und das Medium anschließend als Überstand aus dem Gemisch von Epithelzellen und Stromazellen entfernt wird.

Das Kultivieren unter epithelialer Induktion wird besonders bevorzugt mittels eines konditionierten Mediums durchgeführt, wobei das konditionierte Medium mittels humaner oder tierischer Epithelzellen, insbesondere Urothelzellen, konditioniert worden ist. Ein konditionierte Medium kann erhalten werden, indem ein nicht-konditioniertes Medium mit humanen oder tierischen Epithelzellen kultiviert und das Medium anschließend als Überstand aus dem Epithelzellen entfernt wird. Mikrovaskulären Endothelzellen können aus dem stromalen Gewebe des zu rekonstruierenden Organs gewonnen werden (nicht erfindungsgemäß). Das kann geschehen, indem
(i) das stromale Gewebe mittels einer Kollagenase digeriert wird;
(ii) aus dem in Schritt (i) erhaltenen Gemisch die mikrovaskulären Endothelzellen unter Verwendung von paramagnetischen Lectin- oder Antikörper-gekoppelten Teilchen abgetrennt werden, wobei die Antikörper monoklonale Antikörper für das Blutplättchen-Endothelzell-Adhäsionsmolekül-1 (PECAM 1) oder CD105 sind;
(iii) die so erhaltenen mikrovaskulären Endothelzellen vermehrt werden; und
(iv) aus dem in Schritt (iii) erhaltenen Gemisch die mikrovaskulären Endothelzellen unter Verwendung von paramagnetischen Lectin- oder Antikörper-gekoppelten Teilchen abgetrennt werden, wobei die Antikörper monoklonale Antikörper für das Blutplättchen-Endothelzell-Adhäsionsmolekül-1 (PECAM 1) oder CD105 sind und wobei die so erhaltenen mikrovaskulären Endothelzellen ein Gemisch mit einer Reinheit von zumindest 95 %, bezogen auf die Zahl aller separierten Zellen, ist.

Der in Schritt (ii) verbliebene Rest des Gemisches wird besonders bevorzugt zur Herstellung des konditionierten Mediums verwendet. Alternativ kann der Rest des Gemisches auf der prävaskulariserten Membran im Anschluß an Schritt (c) mit Urothelzellen kultiviert werden und somit ein komplexes vitales Gewebetransplantatkonstrukt bilden.

Die Erfindung bietet die Möglichkeit zur Rekonstruktion der unteren Harnwege mit den erfindungsgemäßen prävaskularisierten biologischen Matrizes, bei denen die Gewebegeneration *in vitro* begonnen wird, wobei gleichzeitig die Proliferation von mikrovaskulären Blasen-Endothelzellen beschleunigt und deren Mikrogefäßbildung verbessert wird. Das erfindungsgemäße Gewebetransplantatkonstrukt kann neben den mikroväskuläre Endothelzellen weitere, gewebespezifische Zelle, wie epitheliale Zellen und stromale Zellen. beinhalten.

Die vorteilhaften Ergebnisse der Erfindungen basieren auf folgenden Erkenntnissen der Erfinder: Die Angiogenese ist ein komplexes Verfahren, das eine extrazelluäre Matrix (ECM) und vaskuläre Endothelzellen einbezieht und durch verschiedene angiogene Faktoren reguliert wird. Die Fähigkeit zur Bildung eines kapillaren/röhrchenförmigen Netzwerkes ist eine spezielle Funktion von Endothelzellen (EC) und erfordert eine spezielle Kaskade von Vorgängen, bestehend aus Endothelzelleninvasion, Migration, Proliferation, Röhrchenbildung und Anastomose zwischen den Strukturen (Cameliet et al., 2000; Yancopoulos et al., 2000; Blau et äl., 2001; Ferrara et al., 1999; Keshet et al., 1999). Auf diese Weise werden Endothelzellen durch die Signale aus der Mikroumgebung der sie umgebenden Matrix geleitet (Berthod et al., 2006).

Um die Mikrogefäßbildung und ihre Aufrechterhaltung *in vitro* zu unterstützen, wird vorzugsweise eine azelluläre Harnblasen- oder Harnleiter-Matrix verwendet, die die Hauptkomponenten von Blasen bzw. Harnleiter-Interstitium als physiologische Matrix für die Endothelzellinvasion und -differenzierung umfassen. Ebenso bevorzugt können andere biologische azelluläre Matrizes verwendet werden, die Kollagen als Hauptkomponente umfassen, welche ebenso die Hauptkomponente des Interstitiums ist. Die Tatsache, daß die mikrovaskuläre Strukturbildung innerhalb aller Matrizes beobachtet wurde, zeigt, daß die Fähigkeit von humanen mikrovaskulären Endothelzellen nicht nur auf einen Typ der biologischen Matrix beschränkt ist.

Endothelzellen sind ebenso dafür bekannt, durch Signale aus anderen Zellen ihrer Mikröumgebung aktiviert zu werden (Berthod et al., 2006; Velasquez et al., 2002). Stromazellen (Fibroblasten und Glattmuskelzellen) werden oftmals an Endothelzellen angelagert, was zur Morphogenese und endgültigen Differenzierung zu dem kapillaren/röhrchenförmigen Phänotyp beiträgt. Stromazellen und speziell Fibroblasten stehen mit der Produktion von Matrixproteinen, die als Gerüst zur Gefäßanordnung und anderer Organstrukturen dienen, in Verbindung. Diese Zellen sind ebenso eine reiche Quelle an angiogenen Wachstumsfaktoren (Honorati et al., 2005), um die Endothelzellproliferation und -differenzierung zu leiten (Erdag et al., 2004; Hudon et al. 2003) und mit der Stabilisierung von Blutgefäßen *in vitro* in Verbindung gebracht zu werden (Velazquez et al., 2002). Ferner ist bekannt, daß epitheliale Zellen Wachstumsfaktoren sezernieren, die eine Gefäßbildung induzieren (Thompson et al., 2006)

Darland et al., 2001, beobachteten, daß Stromazellen, wie differenzierte Perizyten in Kultur mit Endothelzellen Wachstumsfaktoren erzeugen, die das Überleben und/oder die Stabilisierung von Endothelzellen unterstützen.

Adulte Knochenmarksmesenchymstammzellen sind multipotente und stark proliferierende Zellen, die unterschiedliche Wachstumsfaktoren freisetzen können (Tang et al., 2004), die das Überleben und/oder die Stabilisierung von Endothelzellen unterstützen (Markowicz et al., 2005). Diese Zellen sind bekannt dafür, eine lokale Umgebung bereitzustellen, die den Einwuchs von blutgefäßen in eine defekte Stelle unterstützen (Gruber et al., 2005).

In der vorliegenden Erfindung ist die Induktionswirkung von Blasenströmazellen, Knochenmarksstroma-Prögenitorzellen und Blasenurothelzellen durch konditionierendes Medium untersucht worden (Velazquez et al., 2002; Gruber et al., 2005; Thomson et al., 2006). Für diesen Zweck wurden Überstände von aus dem Knochenmark stammenden Stromazellen, organspezifischen. Blasenstromazellen und organspezfischen Blasenurothelzellen verwendet, um die Proliferation von isolierten mikrovaskulären Blasen-Endothelzellen und dermalen mikrovaskulären Endothelzellen, die an den biologischen Gerüsten angelagert sind, durch die parakrine Funktion der Stromazellen und Urothelzellen zu beschleunigen.

In Versuchen, die der Erfindung zugrunde lagen, wurden zunächst humane mikrovaskuläre Blasen-Endothelzellen oder dermale mikrovaskuläre Endothelzellen als Monoschicht auf den Matrizes kultiviert. Anschließend wurden die kultivierten Zellen mit stroma-konditioniertem oder urthelial-stromal konditioniertem Medium gefüttert. In Kontrollversuchen wurden die kultivierten Zellen mit urothelial-konditioniertem Medium oder mit nicht-konditioniertem Medium gefüttert. Um die Fähigkeit von Stromazellen und Urohtelzellen, die Proliferation, Differenzierung und Stabilisierung von mikrovaskulären Endothelzellen auf azellulären biologisch abbaubaren Materialien zu modulieren, zu bestimmen, wurde die *in vitro-*Angiogenese durch histologische und immunohistochemische Analysen zur Bestimmung der Proliferation, Penetration, kapillaren/röhrchenförmigen Netzwerkbildung und des Phänotyps bewertet. Es gibt drei angiogene Parameter, die in der Literatur für die Untersuchung der röhrchenförmigen Netzwerkbildung vorgeschlagen worden sind: Kapillarlänge, Anzahl an Kapillaren und relativer Kapillarbereich. In diesen durchgeführten Versuchen wurde die Kapillarlänge in einem Assay ermittelt (Watanable et al., 2005). Dabei wurde die Länge der Kapillaren in jeweils drei histologischen Längsschnitten von drei verschiedenen Konstrukten der Zellen, gewonnen aus jeweils einer Harnblase, bei lichtmikroskopischen Analysen bestimmt.

Histologische und immunohistochemische Untersuchungen von mikrovaskulären Endothelzellen auf dreidimensionalen biologischen Matrizes, die mit Stromazellen und Gemischen aus Stroma- und Urothelzellen konditioniert wurden, zeigten aktivierte invasive Zellen, die ihre perizelluläre Matrix abbauten, während ihr differenzierter Phänotyp erhalten wurde. Sobald diese Zellen in die dreidimensionale extrazelluäre Matrix freigesetzt wurden, begannen sie zu anderen Zellen hin zu migrieren und richteten sich zu Endothelzellsträngen aus. Die Mikroröhrchenbildungen wuchsen aufeinander zu und bildeten Anastomosen mit anderen Mikroröhrchen unter parakrinen Zytokinsignalen von Stromazellen und Gemischen aus Stroma- und Urothelzellen. Im Vergleich zu diesem System zeigten die Kulturen, die mit nicht-konditionierten Medien oder nur urothelial-konditioniertem Medium beschickt wurden, nur eine geringe Erhöhung der Anzahl an Endothelstrukturen, eine kleinere Oberfläche, die durch Endothelzellen bedeckt wurde, und einen geringeren Prozentsatz an gefäßartigen Strukturen mit Lumen im Vergleich zu stroma-konditionierten Kulturen und urothelial-stromal konditioniertenm Kulturen.

Der Proliferationsassay mit KI-67 zeigte, daß in nicht-konditionierten Kultursystemen oder in nur urothelialkonditionierten Kultursystemen sowie in urothelialkonditionierten die Endothelzellen in einem Zeitraum von ein bis zwei Wochen zum Großteil zwei Wochen latent waren. In stromal- oder urothelial-stromal-konditionierten Kultursystemen blieben die Endothelzellen proliferierend für mindestens vier Wochen, was Konstrukte mit eine höheren Zelldichte als bei Konstrukten in nicht-konditionierten oder nur urothelial-konditionierten Kultursystemen ergab.

Der Assay zur Bestimmung der kapillaren/röhrchenförmigen Netzwerkbildung zeigte ebenso längere Kapillarbildungen pro Feld in den stromal- oder urothelial-stromal konditionierten Systemen.

Somit stellen die Stromazellen sowie Gemisch aus Stroma- und Urothelzellen in ihrem Überstand Faktoren bereit, die die Invasion von Endothelzellen und die Bildung von Mikroröhrchenstrukturen stimulieren können. Diese Ergebnisse lassen darauf schließen, daß Stromazellen oder Gemische aus Stroma- und Urothelzellen kritische Signale mit mitogenen und motogenen endothellialen Wirkungen bereitstellen, was den Aufbau von Röhrchenstrukturen aus Endothelzellen unterstützt.

Die Entwicklung von kapillaren mikrovaskulären Strukturen erfordert nicht nur Stromazellen oder ein Gemisch aus Stromaund Urothelzellen, sondern ebenso die Gegenwart einer extrazellulärer Matrix (ECM). Tatsächlich bilden Endothelzellen keine Röhrchenstrukturen unter Stromainduktion oder urothelial-stromaler Induktion in zweidimensionalen konventionellen Kulturschalen. ECM-Komponenten der biologischen Membranen können sowohl als Gerüst als auch als Induktor für Endothelzellen fungieren. Offensichtlich wird synergistisch zu der Aktivierung der Endothelzellen, indem sie der extrazellulären Matrix ausgesetzt werden, durch Stromazellen oder Gemische aus Stroma- und Urothelzellen Signale zur Beschleunigung der Migration, des Überlebens und der Differenzierung der Endothelzellen zur echten Kapillarmorphogenese bereitgestellt.

Die Tatsache, daß eine erhöhte Proliferation und Differenzierung von Endothelzellen durch Medien, die mittels Blasenstromazellen, Knochenmarksstroma-Progenitorzellen oder einem Gemisch aus Urothelzellen und Stromazellen konditioniert wurden, auf keine Organspezifität hinsichtlich der durch die Stromazellen oder die Gemische aus Stroma- und Urothelzellen vermittelten Wirkungen auf die kapillar-artige Morphologie schließen läßt und impliziert eine kritische Rolle von Stromazellen oder des Gemisches aus Urothelzellen und Stromazellen bei der Kontrolle des Verhaltens der Endothelzelle.

Der vaskuläre Endothelwachstumsfaktor (VEGF) stellt einen der wirksamsten Endothelzellmitogene dar (Pepper et al., 1992 - Lazarous et al., 1997). Er ist unter den Faktoren, die die Expression von Matrix-Metalloproteinasen, Proliferation, Migration und Röhrchenbildung von isolierten Endothelzellen *in vitro* und die Entwicklung von Blutgefäßen *in vivo* stimulieren (Gruber et al., 2005).

Um die Gegenwart dieses vaskulogenen und angiogenen Schlüsselfaktors in den konditionierten Medien zu bestimmen, wurde die Konzentration des vaskulären Endothelwachstumsfaktors (VEGF) in ELISA-Tests untersucht. Die Ergebnisse zeigten nachweisbare Konzentrationen von VEGF, die darauf schließen lassen, daß meßbare Konzentrationen dieses Zytokins durch Stromazellen sekretiert werden. Die erfindungsgemäßen Konstrukte basieren somit insbesondere auf der Kapazität der Wachstumsfaktorexpression durch Stromazellen und Gemische aus Stroma- und Urothelzellen. Bezüglich dieses Wachstumsfaktors produzierten die Urothelzellen höhere Werte. Die Mischkulturen aus. Urothelzellen und Stromazellen bildeten dieses Zytokin am stärksten, wobei die höchste Konzentration von VEGF in dem mit dem Gemisch aus Stroma- und Urothelzellen konditioniertem Medium zu messen war.

Die Tatsache, daß die Proliferation und die Röhrchen-Kapillarbildung durch ein konditioniertes Medium mit nur Urothelzellen (trotz Produktion von VEGF durch diese Zellen) wenig induziert wurden, deutet darauf hin, daß die zusätzliche Anwesenheit der Stromazellen für die Induktion der Endothelzellen vorteilhaft ist. Die induzierende Wirkung von Stromazellen ihrerseits kann somit durch die Urothelzellen erhöht werden.

Andererseits führte nach der Kultivierung der mikrovaskulären Endothelzellen als Einzelschicht auf den azellulären Matrizes die weitere Kultivierung ohne konditioniertes Medium ( d. h. ohne stromale oder urothelial-stromale Induktion) oder nur mit urothelial-konditioniertem Medium zur Ablösung von Zellen aus der Einzelschicht und die Invasion dieser Zellen in die Matrix, aber die Invasion der unterliegenden Matrix war nicht so hoch wie unter konditionierten Bedingungen mit Stromazellen oder mit einem Gemisch aus Stroma- und Urothelzellen. Außerdem ordneten sich die Endothelzellen nur in wenigen polarisierten Strängen mit geringer Verzweigung an, und nur wenige davon wiesen ein vollständig ausgebildetes Lumen auf. Daher ist die azelluäre Matrix für die anfängliche Migration in die untere azelluläre Schicht wesentlich, aber nicht ausreichend für die Förderung der weiteren Lokomotion und für die Bildung von reifen Röhrchenstrukturen innerhalb der azellulären Matrix. Diese Ergebnisse zeigen, daß die stromale Induktion oder die urothelial-stromale Induktion für die adäquate Migration und Invasion und für die Bildung von reifen luminalen Strukturen notwendig ist.

Zusammengenommen zeigen die *in vitro* Ergebnisse, auf denen die Erfindung basiert, daß die Prävaskularisation von biologischen azellulären Matrizes durch isolierte autologe mikrovaskuläre Endothelzellen, geerntet aus einer kleinen Blasenbiopsie oder Haut, und induziert durch Blasenstromazellen, Knochenmarksstroma-Progenitorzellen oder einem Gemisch aus Blasenurothelzellen und Blasenstromazellen, eine erfolgreiche Möglichkeit zur Behandlung der Mängel der unteren Harnwege darstellt.

Diese Erkenntnisse sind jedoch nicht auf die unteren Harnwege, insbesondere die Harnblase beschränkt, sondern können auch auf andere Organe, insbesondere Weichteilorgane, übertragen werden.

Nachstehend wird die Erfindung anhand von Beispielen unter Bezugnahme auf die Zeichnungen näher erläutert. Es zeigen
- Fig. 1: ein Säulendiagramm, daß die VEGF-Konzentration in konditionierten und nicht-konditionierten Medien zeigt;
- Fig. 2: ein Säulendiagramm, daß die Zellpopulation von mi- krovaskulären Endothelzellen nach Kultivierung mit unterschiedlichen konditionierten Medien innerhalb der Membran zeigt;
- Fig. 3: ein Säulendiagramm, daß die Länge mikrovaskulären Strukturen in Gewebetransplantatkonstrukten zeigt, deren mikrovaskuläre Endothelzellen mit unter- schiedlichen Kulturmedien kultiviert worden waren;
- Fig. 4A: eine histologische Aufnahme zur Charakterisierung einer in-vitro-Kultur gesäter mikrovaskulärer Bla- sen-Endothelzellen auf der Oberfläche einer azellu- lären Matrix unter stromaler bzw. urothelial- stromaler Induktion am Tag 14, wobei die Blasen- Endothelzellen bei Induktion mittels stromaler bzw. urothelial-stromal löslicher Faktoren von der Ober- fläche der Matrix losgelöst und in das Gerüst der Matrix eingedrungen sind. Zunächst wird ein Strang gebildet, der von Zellen ausgekleidet ist. Allmäh- lich kann ein vollständig ausgebildetes Lumen er- kannt werden (Pfeil);
- Fix. 48: eine histologische Aufnahme zur Charakterisierung einer in-vitro-Kultur gesäter mikrovaskulärer Bla- sen-Endothelzellen auf der Oberfläche einer azellulären Matrix unter stromaler bzw. urothelial- stromaler Induktion am Tag 14, die ein Röhrchen im Inneren des Gerüsts unter Induktion bildeten;

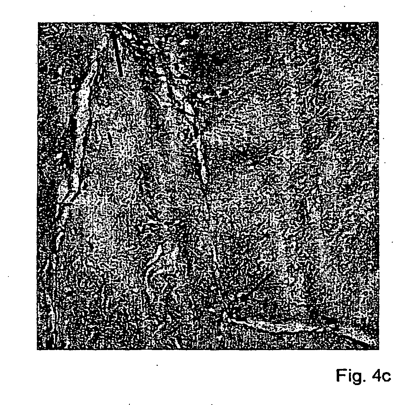

- Fig. 4C: eine histologische Aufnahme zur Charakterisierung einer in-vitro-Kultur gesäter mikrovaskulärer Bla- sen-Endothelzellen auf der Oberfläche einer azellu- lären Matrix unter Induktion mit einem stromal bzw. urothelial-stromal konditioniertem Medium am Tag 21, wobei neue Verzweigungen (Pfeile) des Röhrchens mit dem vollständig ausgebildeten Lumen, das mit mikrovaskulärer Blasen-Endothelzellen ausgekleidet ist, zu erkennen sind; und

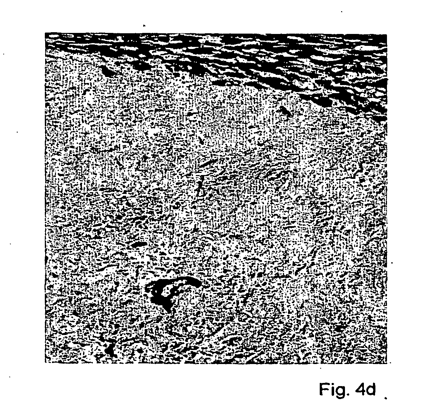

- Fig. 4D: eine immunohistochemische Aufnahme zur Charakteri- sierung einer in-vitro-Kultur gesäter mikrovaskulä- rer auf der Oberfläche einer azellulären Matrix un- ter Induktion mit einem stromal bzw. urothelial- stromal konditioniertem Medium am Tag 28, wobei die luminale Struktur durch die Blasen-Endothelzellen ausgekleidet ist und wobei eine positive Exprimie- rung des Endothelmarkers von-Willebrand-Faktor er- kennbar ist. Die mehrfache Schicht von gesäten mi- krovaskulären Blasen-Endothelzellen auf der Matrix- oberfläche exprimierte ebenso positiv den genannten Antikörper.

### Beispiele

Die folgenden Beispiele veranschaulichen das erfindungsgemäße Verfahren zur Herstellung von Gewebetransplantatkonstrukturen für untere Harnorgane.

Soweit nicht gesondert angegeben haben die verwendeten Abkürzungen folgende Bedeutung:

| | |
|---|---|
| b-FGF | b-Fibroblast-Wachstumsfaktor |
| DMEM | Dulbecco's modifiziertes Eagle's Medium |
| UEA-1 | Ulex Europeaus Agglutinin 1 Lectin |

### Materialien und Verfahren:

### (a) Herstellung von azellulären Matrizes

Humane oder porzine Harnblasen und Harnblasensubmukosa, Harnröhre, Dermis und Dünndärme wurden mit. Phosphat-gepufferter Kochsalzlösung (PBA) (Invitrogen, Deutschland) gewaschen und einer Behandlung mit Triton X 1 % für 24 bis 48 Stunden unter vorsichtigem Schütteln bei 37 °C unterzogen. Die Azellularität der Matrizes wurde histologisch überprüft.

### (b) Isolation, Kultivierung und Charakterisierung von humanen mikrovaskulären Blasen-Endothelzellen und dermalen mikrovaskulären Endothelzellen

Zur Isolierung von mikrovaskulären Blasen-Endothelzellen wurde humanes Blasengewebe von vier Patienten erhalten, die einer offenen Blasenoperation unterzogen wurden. Urothel wurde mikrodisseziert, und das Stromagewebe zur Enzymspaltung zerkleinert und mit 1 mg/ml Kollagenase Typ II (Worthington Biochemical Corporation, USA)/Dispase (0,05 mg/ml) (Invitrogen)/16 µg/ml Desoxyribonuclease Typ I (Boeringer Mannheim GmbH, Deutschland) in DMEM-Medium, enthaltend 0,2 % Serumalbumin (Sigma Aldrich, Deutschland), für 2 Stunden bei 37 °C unter kontinuierlichem Rühren digeriert. Das digerierte Material wurde bei 2000 rpm zentrifugiert und die Zellsuspension wurde gesammelt, in DMEM-Medium verdünnt und durch ein Zellfiltriergerät mit einem 40 µm Nylonsieb (BD Labware, Deutschland) filtriert. Das filtrierte Material wurde in DMEM-Medium (Invitrogen, Deutschland), enthaltend 2,5 % humanes Serum, verdünnt. Paramagnetische Polystyrol-Partikel (Dynabeads) (Dynal, Deutschland) wurden mit Lektin UEA-1 (Sigma Aldrich, Deutschland) oder mit monoklonalem Antikörper für Blutplättchen-Endothelzell-Adhäsionsmolekül-1 (PECAM 1; CD 31) (Dakocytomation, Dänemark) durch Inkubieren von 10⁷ Dynabeads mit dem Lektin oder dem Antikörper CD 31 für 24 Stunden bei Raumtemperatur unter Überkopfrotation, gemäß den Herstellerhinweisen gekoppelt. Die Dynabeads wurden unter Verwendung einer Vorrichtung zur Konzentration der Magnetteilchen gesammelt und dreimal in PBS/0,1 % Serumalbumin gewaschen. Mikrovaskuläre Blasen-Endothelzellen wurden gezählt und bei 4 °C für 10 min mit den UEA 1- oder CD 31-beschichteten Dynabeads unter Überkopfrotation inkubiert. Die positiv selektierten Zellen wurden anschließend aus der Mischzellpopulation unter Verwendung einer Vorrichtung zur Konzentration der Magnetteilchen (Dynabeads-Endothelzellen-Verhältnis 10 : 1) entfernt. Die an die Dynabeads angelagerten Zellen (Endothelzellen) wurden rückgewonnen und 10mal in DMEM/2,5 % humanes Serum gewaschen. Die negativ selektierten nicht-angelagerten Zellen (Blasenstromazellen) wurden in PBS gewaschen und in Kulturflaschen bei den unter (c) genannten Bedingungen kultiviert. Die gereinigten Endothelzellen wurden in Kulturmedium resuspendiert, wobei 10⁴ Zellen/cm² in Kulturschalen gesät wurden. Das Medium wurde alle 2 bis 3 Tage gewechselt. Die Zellen wurden bei 70 % Konfluenz passagiert. Nach der zweiten Passage wurden die Zellen wieder mit Lektin UEA-1- oder CD 31-gekoppeltem Dynabeads gereinigt. Der Endothelphänotyp der Zellen wurde durch immunohistochemische Färbung unter Verwendung von von-Willebrand-Faktor und CD-34-Antikörper (beide Dako) bestätigt. Die Zellen konnten ebenso unter Anwendung von Dynabeads getrennt werden, an die CD105 bzw. CD31 (Miltenyi, Deutschland) gekoppelt war.

Die so erhalten mikrovaskulären Blasen-Endothelzellen der dritten bis fünften Passage wurden in flüssigem Stickstoff gelagert und für die Experimente verwendet.

Zur Isolierung von humanen dermalen mikrovaskulären Endothelzellen wurde humane Vorhaut von Patienten, die einer Beschneidung unterzogen worden waren, mehrfach mit PBS gewaschen. Die Dermis wurde von subkutanen Fett mechanisch mittels Mikroscheren getrennt. Das Gewebe wurde zur Trennung der Dermis von der Epidermis in 0,25 % Dispase (Boeringer Mannheim, Deutschland) 2 Stunden inkubiert. Die Dermis wurde dann zerkleinert und 30 min in Trypsin (0,05 %)/EDTA (0,01 %) (Invitrogen, Deutschland) inkubiert. Die digerierte Zellsuspension wurden durch ein Nylonsieb (40 µm Porengröße) filtriert und zentrifugiert. Die Zellpellets wurden in EGM-2 (Cambrex, Deutschland) verdünnt bis zur Subkonfluenz. Die Zellen wurden anschließend aus den Kulturschalen entnommen, und die dermalen mikrovaskulären Endothelzellen positiv selektiert unter Verwendung von Dynabeads, an die CD-31 gekoppelt war. Die Zellen der dritten Passage wurden einem zweiten Trennverfahren unterzogen. Der endotheliale Phänotyp der Zellen wurde durch immunhistochemisches Färben für den von-Willebrand-Faktor und CD34 betätigt. Die Zellen der dritten bis fünften Passage wurden im flüssigem Stickstoff gelagert und für weitere Experimente verwendet.

### (c) Isolation und Kultivierung von humanen adulten Blasenstromazellen und Knochenmarksstroma-Progenitorzellen

Die übrigen (negativ selektierten) Blasenstromazellen, die nicht an die Dynabeads gekoppelt wurden, wurden in PBS gewaschen und anschließend in DMEM (Invitrogen, Deutschland) mit zusätzlichem Serum und Penicillin-Streptomycin kultiviert. Die immunohistochemische Charakterisierung wurde mit Antikörpern gegen α-Glattmuskel-Aktin, Vimentin und Panzytokeratin beurteilt.

Knochenmarksstroma-Progenitorzellen wurden aus dem Knochenmarksmaterial, erhalten aus Darmbeinkammknochen von sechs gesunden erwachsenen Patienten, durch Nadelaspiration gewonnen und in einem heparinisierten 50 ml Teströhrchen gesammelt. Aspiriertes Material wurde mit einem gleichen Volumen an DMEM/10 % Serum gemischt. Die Zellsuspension wurde auf die Oberseite von 10 ml Ficoll-Paque (Amersham Pharmacia Biotech AB, Schweden) geschichtet und bei 400 g für 35 min bei 4 °C zentrifugiert. Mononukleäre Zellen wurden aus der Interphase gesammelt, durch eine Zellfiltriervorrichtung mit einem 100-µm-Nylonsieb filtriert (Becton Dickinson; Deutschland) und in DMEM, ergänzt mit humanem Serum und Penicillin/Streptomycin, bei einer Konzentration von 10⁶ Zellen/cm² resuspendiert. Die Zellen wurden mittels. Durchflußzytometrie charakterisiert. Für diesen Zweck wurden Knochenmarksstroma-Progenitorzellen trypsinisiert, mit PBS gewaschen und mit Antikörpern gegen CD34, CD45, CD44, CD73, CD90 (alle BectonDickinson) und CD133 (Miltenyi Biotech; Deutschland) inkubiert. Die Analyse wurde mit einem FACScalibur-Durchflußzytometer (Becton Dickinson) durchgeführt. Die Zellen wurden zur Konfluenz expandiert wobei das Kulturmedium alle 3 bis 4 Tage gewechselt wurde.

Blasenstromazellen und Knochenmarksstroma-Progenitorzellen der ersten fünf Passagen wurden in flüssigem Stickstoff gelagert und für die Experimente verwendet.

### (d) Isolierung und Kultivierung von Urothelzellen und Urothel-Blasenstromazellen

Zur Kultivierung von Blasenurothelzellen wurde Blasenmukosa, das mittels Mikrodissektion aus Blasenbiopsien erhalten worden war, in schmale Stücke geschnitten, in Kollagenase Typ II 1 mg/ml 2 Stunden digeriert, bei 2000 rpm 5 min zentrufugiert und in 25-ml-Kulturschalen mit Keratinozyt-freiem Serum (Cambrex) kultiviert.

Um ein Gemisch aus Blasenurothelzellen und Blasenstromazellen zu erhalten, wurde Blasenbiopsien, die aus Blasenurothel und Blasenstroma bestanden) in kleine Stücke geschnitten und wie oben beschrieben digeriert und gesammelt. Zellen der ersten fünf Passagen wurden in flüssigem Stickstoff gelagert und für die weiteren Versuche verwendet.

### (e) Herstellung von Medium, die mit Blasenstromazellen, Knochenmarksstroma -Progenitorzellen, Urothelzellen und Urothel-Blasenstromazellen konditioniert wurden:

*Jeweils unabhängig von einander wurden Blasenstromazellen, Knochenmarksstroma-Progenitorzellen, Urothelzellen und Urothel-Blasenstromazellen* bis zur Konfluenz vermehrt, bevor das Medium auf 20 ml DMEM, ergänzt mit Serum und Antibiotika, für 72 h geändert wurde. Die konditionierten Medien wurden einer sterilen Filtration unterzogen. Es wurde b-FGF 20 ng/ml in jedes konditionierte Medium supplementiert. Anschließend wurden die Medien in Aliquoten geteilt und bei -80 °C gelagert.

ELISA wurde verwendet, um die Konzentration des vaskulären endothelialen Wachstumsfaktors (VEGF) in den konditionierten Medien zu bestimmen. Zur Messung der Zytokinkonzentration wurde ein quantitativer ELISA unter Verwendung von kommerziellen Kits für den vaskulo-endothelialen Wachstumsfaktor entwickelt (R&D-Systeme). Für diese Tests wurden die konditionierten Medien nach 72 Stunden Kultivierung gesammelt, bei 2000 rpm für 10 Minuten zentrifugiert und durch einen 0,3-µm-Filter geleitet. Alle Assays wurden dreifach auf Mikrotiterplatten durchgeführt.

### (f) Kultivierung mikrovaskulärer Blasen-Endothelzellen und dermalen mikrovaskulären Endothelzellen auf biologischen Gerüsten

Gemäß (a) gewonnene mikrovaskuläre Blasen-Endothelzellen oder dermale mikrovaskulären Endothelzellen wurden in Kulturschalen mit Trypsin/EDTA kultiviert und bei 70 % Konfluenz entnommen, gezählt und zentrifugiert, um Pellets der gewünschten Anzahl an Zellen zu erhalten. Die Zellen wurden in Kulturmedium resuspendiert und homogen auf der oberen Oberfläche der Matrizes bei einer Endkonzentration von 10⁴/cm² verteilt. Die Zellkultivierung wurde für 28 Tage bei 37 °C durchgeführt. Die Zellen wurden kultiviert mit
(a) DMEM, ergänzt mit Serum und b-FGF (Kontrollgruppe a); oder
(b) Blasenstroma-konditioniertem Medium, ergänzt mit b-FGF (Kulturgruppe b); oder
(c) Knochenmarksstromapräkursorzellen-konditioniertem Medium, ergänzt mit b-FGF (Kulturgruppe c); oder
(d) Urothel-konditioniertem Medium, ergänzt mit b-FGF (Kontrollgruppe d); oder
(e) Urothel-Blasenstroma-konditioniertem Medium, ergänzt mit b-FGF (Kulturgruppe e).

Alle Experimente wurden zumindest im Doppel durchgeführt und unabhängig voneinander dreimal wiederholt.

### (g) Mikroskopische Analysen von kultivierten mikrovaskulären Blasen-Endothelzellen oder dermalen mikrovaskulären Endothelzellen auf den biologischen Gerüsten

Am Ende jedes Experiments wurden Formalin-fixierte biologische Gerüste mit reihenweise erhöhenden Konzentrationen an Alkohol dehydratisiert, in Paraffin eingebettet und in Abschnitte mit 6 mm Breite geschnitten. Die Zusammensetzung der Konstrukte wurde durch Hämatoxylin-Eosin (HE) und Immunohistochemie unter Verwendung von anti-humanem von-Willebrand-Faktor, Endothelzellen-spezifisches Lektin UEA-1, anti-humanem CD34 und antihumanen CD31 (PECAM-1) analysiert und unter Verwendung eines Phasenkontrastmikroskops (Zeiss, Deutschland) bewertet. Die Zellproliferation wurde mit dem Proliferationsmarker K167 bewertet. Es gibt drei Parameter, die man zur Beurteilung der Angiogenese verwenden kann: Kapillarlänge, Anzahl an Kapillaren und relativer Kapillarbereich. Wir bewerteten die Kapillarlänge in drei.verschiedenen Längsschnitten von jeder Probe mittels des Assays (Watanable et al., 2005). Dabei wurde die Länge der Kapillaren in jeweils drei histologischen Längsschnitten von drei verschiedenen Konstrukten von Zellen, gewonnen aus jeweils einer Harnblase bei lichtmikroskopischen Analysen bestimmt. Für diesen Zweck wurde die Länge von kapillaren/röhrchenförmigen Strukturen in histologischen Abschnitten innerhalb der Matrix durch manuelle Messung der Röhrchen quantifiziert. Die Zelldichten von repräsentativen histologischen Abschnitten wurden in derselben Weise quantifiziert.

### Ergebnisse

### Isolation und Kultivierung von humanen Blasen-mikrovaskulären Endothelzellen und dermalen mikrovaskulären Endothelzellen

Zunächst mußte ein Selektionsverfahren entwickelt werden, um primäre Kulturen von mikrovaskulären Blasen-Endothelzellen aus Blasenstromagewebe zu isolieren und herzustellen, welches in erster Linie aus Glattmuskelzellen, Fibroblasten und Endothelzellen besteht (siehe den vorstehenden Abschnitt (a)). Dazu wurden Le.ktin-beschichtete oder Antikörper-beschichtete Dynabead-Partikel verwendet. Es wurde festgestellt, daß eine zusätzliche Dynabead-Reinigung während der ersten fünf Passagen notwendig war, um > 95 % reine Kulturen von mikrovaskulären Blasen-Endothelzellen herzustellen. Die angelagerten Dynabeads wurden innerhalb der ersten zwei Passagen ausgewaschen und interferierten nicht mit dem Wachstum oder Überleben der mikrovaskulären Endothelzellen. Die Separation der dermalen mikrovaskulären Endothelzellen ergab eine Kultur, die 80 % mikrovaskuläre Endothelzellen enthielt. Eine zweite Separation der dritten Passage ergab eine Reinheit von über 90 %. Die Morphologie von primären mikrovaskulären Endothelzellen der ersten Passage zeigte etwas Variabilität in der Größe und Form. Mit zunehmender Passagenzahl zeigte die Zellpopulation eine homologe Morphologie. Kultivierte humane mikrovaskuläre Blasen-Endothelzellen zeigten die charakteristischen Merkmale von Endothelzellen. Sie exprimierten Faktor VIII-bezogenes Antigen und CD 34.

### Isolation, Kultivierung und Charakterisierung von Blasenstromazellen, Knochenmarksstroma-Progenitorzellen und Blasenurothel-Stromazellen.

Primäre Kulturen von Blasenstromazellen und Knochenmarksstroma-Progenitorzellen wurden erfolgreich aus Blasenstroma und aspiriertem Knochenmark hergestellt. Eine Mischkultur aus Blasenurothelzellen und Blasenstromazellen war auch erfolgreich und enthielt Urothelzellen, Glattmuskelzellen rund Fibroblasten. Reine Urothelzellen wurden aus Harnblasenmukosa erfolgreich gewonnen. Isolierte Zellen begannen zu haften und wuchsen während der ersten 2 Tage. Sie blieben für 3 bis 5 Tage inaktiv; sie begannen dann sich schnell zu vermehren. Kulturen von Blasenstromazellen zeigten eine einheitliche Morphologie mit spindelförmigen Zellen. In der primären Kultur (P⁰) zeigten Knochenmarksstroma-Progenitorzellen etwas Variabilität in ihrer Größe und Form; bestehend aus drei unterschiedlichen Morphologien. Nach der ersten Passage zeigten diese Zellen eine homogene, spindelförmige Morphologie. Blasenstromazellen und Knochenmarksstromapräkursorzellen zeigten einander ähnliche proliferative Potentiale und Wachstumsmuster. In beiden Kulturen wurde die Konfluenz nach ungefähr 10 Tagen.erhalten. In den letzten Passagen (>P⁵) begannen die spindelförmigen Zellen, eine verbreiterte, flache Morphologie zu zeigen. Deshalb wurden die konditionierten Medien nur auf Zellen der Passagen 1 bis 5 (P¹ bis P⁵) geerntet.

Die kultivierten Urothelzellen zeigten ihre typische epitheliale Morphologie, die unter serumfreier Konditionierung einheitlich war. Die Mischkultur aus Urothelzellen und Blasenstromazellen zeigte gleichzeitig Zellen mit urothelialem Phänotyp und stromalem spindelförmigen Phänotyp. Urohtelzellkulturen und Urothel-Blasenstromazellkulturen wurden bei Subkonfluenz passagiert. Hier wurden ebenfalls die Zellen der ersten fünf Passagen zur Herstellung der konditionierten Medien benutzt.

Knochenmarksstroma-Progenitorzellen wurden mit Durchflußzytometrie für die Gegenwart oder Abwesenheit von charakteristischen hämatopöetischen Markern getestet. Sie exprimierten typischerweise die Antigene CD105 und CD73. Außerdem exprimierten die Zellen CD90 und CD44. Sie waren für typische lymphozytische Marker CD45 und für die frühen hämatopoetischen Marker CD34 und CD133 negativ.

Immunohistochemische Analysen von Blasenstromazellen zeigten, daß diese Zellen aus zwei Zellpopulationen mit etwa 50 bis 60 % Zellen, die positive Exprimierung für α-Glattmuskel-Actin zeigen, und 40 bis 50 % Vimentin-positive Zellen ohne α-Glattmuskel-Actinexprimierung bestanden. Die Urothelzellkulturen bildeten eine Zellpopulation, die ausschließlich Panzytokeratin exprimierte. Die Urohtel-Blasenstromamischkultur bestand gleichzeitig aus drei Zellpopulationen, nämlich panzytokeratin-positiven Zellen, α-glattmuseklactin-positiven Zellen und vimentin-positiven Zellen. Die letzten beiden Zelltypen färbten sich nicht mit Panzytokeratin-Antikörper.

### ELISA-Test

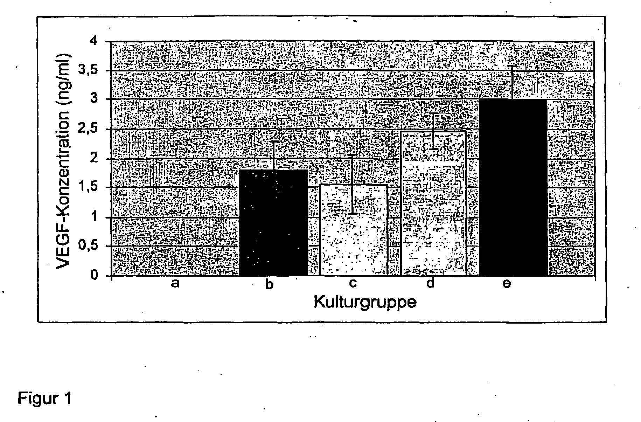

Die Medien, die aus den kultivierten Blasenstromazellen, Knochenmarksstroma-Progenitorzellen, Urothelzellen oder einem Gemisch von Blasenurothelzellen und Blasenstromazellen gewonnen wurden, zeigten im ELISA-Test nachweisbare Konzentrationen von VEGF, wobei.die höchste Konzentration aus der Mischkultur aus Blasenurothelzellen und Blasenstromazellen berechnet wurde (Fig. 1).

### Mikroskopische Analysen von kultivierten mikrovaskulären Blasen-Endothelzellen auf biologischen Gerüsten

Mikroskopische Untersuchungen von kultivierten mikrovaskulären Endothelzellen auf biologischen Gerüsten zeigten die Haftung und das Überleben von mikrovaskulären Endothelzellen auf den biologischen Gerüsten. Nach 24 h begannen die mikrovaskulären Endothelzellen auf der Oberfläche der azellulären Membranen zu migrieren, während sie ihren differenzierten Phänotyp behielten (positive Immunoreaktivität mit von-Willebrand-Faktor, CD31 und CD 34 und Anbindung von UEA-1). Sie bedeckten bis zu 45 cm² der Matrixoberflächen und erreichten die Konfluenz als Einzelschicht innerhalb von zwei Wochen, was eine höhere Migrationskapazität in den konditionierten Systemen (Kulturgruppen b, c und e) zeigt. Sie nahmen zwei unterschiedliche Morphologien mit einem runden Phänotyp und einem verlängerten, länglichen Phänotyp in den konditionierten Medien (Kulturgruppen b, c und e) und meistens runde Morphologie in nicht-konditionierten Systemen (Kontrollgruppe a) und in nur mit Urothelzellen konditionierten Systemen (Kontrollgruppe d) an. Kulturen, die mit konditionierten Stromamedien (Kulturgruppen b, c und e) beschickt wurden, zeigten eine höhere Gesamtzelldichte nach 28 Tagen in Kultur im Vergleich zu nicht-konditionierten Kontrollgerüsten (Kontrollgruppe a). Die Gesamtzelldichte war am höchsten in den Kultursystemen, die mit Urothel-Blasenstromazellen konditioniert worden waren (Kulturgruppe e). Das nur mit Urothelzellen konditionierte Medium (Kontrollgruppe d) zeigte keine erhöhte Zellzahl im Vergleich zu den nicht-konditionierten Systemen. Tatsächlich wurde in konditionierten Kultursystemen (Kulturgruppen b, c und e) die Proliferation der mikrovaskulären Endothelzellen in der Matrix bis zu 28 Tage beobachtet, wie durch Färben für den Proliferationsmarker Ki67 gezeigt wurde, und die Zelldichte erhöhte sich mit der Zeit. Im Vergleich dazu blieben die mikrovaskulären Endothelzellen in den nicht-konditionierten Kultursystemen und in den nur mit Urothelzellen konditionierten Medium (Kulturgruppe d) nur in den ersten 7 bis 14 Tagen proliferierend, aber starben langsam über die letzten zwei bis drei Wochen der Kultur. Die beobachtete Kulturzeit war für alle Gruppen 28 Tage.

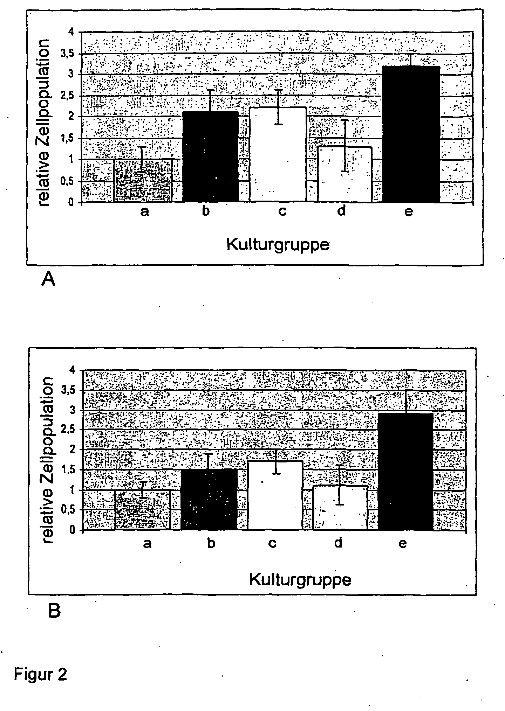

Zwischen dem mit Blasenstromazellen (Kontrollgruppe a) konditionierten Medium und dem mit Knochenmarksstroma-Progenitorzellen konditionierten Medium (Kulturgruppe b) wurden keine signifikanten Unterschiede festgestellt. In diesem beiden Kultursystemen war die Anzahl der mikrovaskulären Blasen-Endothelzellen 2,1- bis 2,2fach höher as in der Kontrollgruppe a (Fig. 2). Die Zahl der dermalen mikrovaskulären Endothelzellen war in diesen beiden Kultursystemen (Kulturgruppen b und c) 1,5- bis 1,7fach höher als in der Kontrollgruppe a. (Fig. 2). In den Kultursystemen mit einem Medium, das mit Urothel-Blasenstromazellen konditioniert worden war (Kulturgruppe e), hatte sich die Anzahl an mikrovaskulären Blasen-Endothelzellen und dermalen mikrovaskulären Endothelzellen auf das 3,2- bzw. 2,9fache erhöht, jeweils verglichen mit der Kontrollgruppe a (Figur 2).

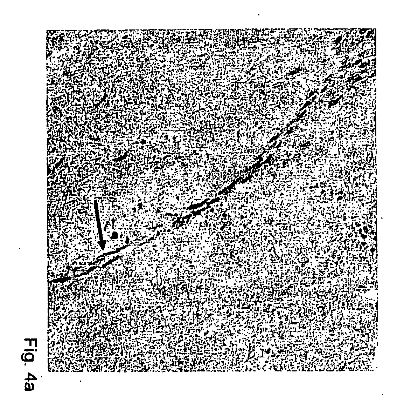

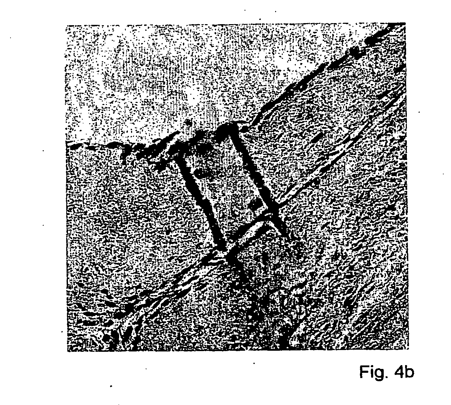

Die Verwendung von konditionierten Medien (Kulturgruppen b, c und e) führte zu einer. Penetration der mikrovaskulären Blasen-Endothelzellen in die Matrizes mit einer Durchdringungstiefe von bis zu 2,3 mm. In diesen konditionierten Systemen wurde überdies die Bildung von Strängen (Röhrchen) (Fig. 4a) und nach 10 bis 14 Tagen die Bildung von Kapillarnetzwerken induziert. Der Grad der Netzwerkbildung war von der Dauer der Kultivierung bis Tag 28 abhängig. In Übereinstimmung mit der kapillarartigen Differenzierung wurden mikrovaskuläre BlasenEndothelzellen bzw. dermale mikrovaskuläre Endothelzelle festgestellt, die sich zu vollständig ausgebildeten kapillaren Lumen vereinigten, die mit einer Monoschicht der mikrovaskulären Endothelzellen ausgekleidet waren (Figuren 4B, 4C und 4D) und positiv für den von-Willebrand-Faktor (Fig. 4D) gefärbt waren.

In Abwesenheit von konditioniertem Medium (Kontrollgruppe a) und in Kultursystemen, die mit nur Urothelzellen konditioniert worden waren (Kontrollgruppe d) zeigten die mikrovaskuläre Blasen-Endothelzellen und die dermalen mikrovaskulären Endothelzellen minimale Penetration in die Matrix und bildeten nur wenig unreife unterbrochene Stränge über den Kulturzeitraum.

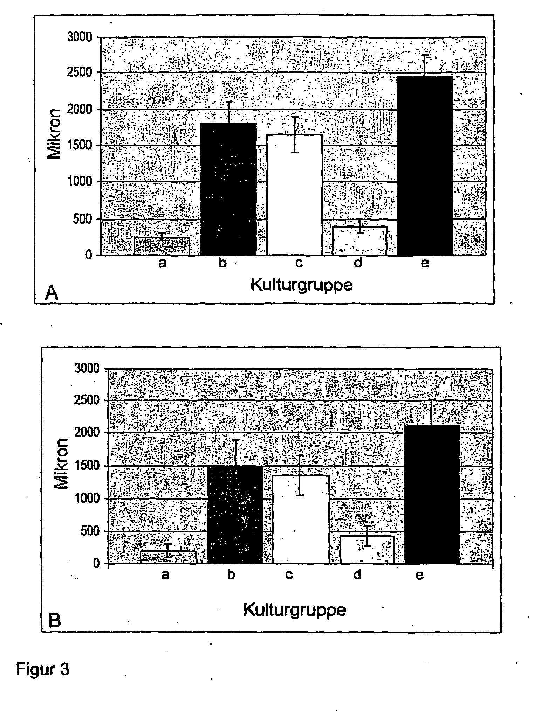

Zur Bewertung der Angiogeneseaktivität wurde die Kapillarlänge in jeweils drei histologische Längsschnitten von drei verschiedenen Konstrukten von Zellen, gewonnen aus jeweils einer Harnblase, bestimmt. Die quantitative Bewertung der dreidimensionalen *in*-*vitro*-Angiogenese wurde durch mikroskopische Messung der Länge der geformten Röhrchen in drei Gesichtsfeldern durchgeführt. Die Werte sind in Fig. 3 dargestellt. Die größte Kapillarlänge zeigten dermale mikrovaskuläre Endothelzellen unter urothel-stromaler Induktion (Gruppe e).

In diesen Kultursystemen erreichten die mikrovaskulären Blasenendothelzellen eine gesamte Kapillarlänge von 240 µm und die dermalen mikrovaskulären Blasenendothelzellen eine gesamte Kapillarlänge von 2100µm (Fig. 3).

In Gegenwart von Stromäzytokinen oder Urothel-Stromazytokinen schienen nicht alle mikrovaskulären Blasen-Endothelzellen bzw. dermalen mikrovaskulären Endothelzellen zur Invasion kompetent zu sein. Die verbliebenen angelagerten mikrovaskulären Blasen-Endothelzellen bzw. dermalen mikrovaskulären Endothelzellen drangen nicht in die Matrix ein, sondern migrierten nur auf der Oberfläche der Matrix und bildeten darauf Multischichten von Zellen, sie stellten dadurch wahrscheinlich eine Subpopulation einer heterogenen Zellpopulation dar.

Obwohl Urothelzellen eine Quelle für die VEGF-Produktion sind, konnte keine induzierende Wirkung von dem nur mit Urothelzellen konditionierten Medium auf mikrovaskuläre Endothelzellen festgestellt werden. Dieses Ergebnis deutet darauf hin, daß die Urothelzellen den zusätzlichen Effekt der Blasenstromazellen benötigen, um die Gefäßbildung *in vitro* zu induzieren. Der synergistische Effekt von Urothelzellen und Stromazellen zeigte den stärksten induzierenden Effekt auf mikrovaskuläre Endothelzellen bezüglich Zellproliferation und Röhrchen-Kapillar-Bildung.

### Literatur

Alberti C, Tizzani A, Piovano M, Greco A. What's in the pipeline about bladder econstructive surgery? Some remarks on the state of the art Int J Artif Organs. 2004; 27(9): 737 - 43.
Atala A. New methods of bladder augmentation. BJU Int. 2000; 85Supp13: 2434; discussion36.
Blau, H. M., and Banfi, A. The well-tempered vessel. Nature Med.2001; 7, 532 - 534
Berthod F, Germain L, Tremblay N, Auger FA.J Cell Physiol. Extracellular matrix deposition by fibroblasts is necessary to promote capillary-like tube formation in vitro.2006 Feb 1.
Carmeliet, P., and Jam, R. K. Angiogenesis in cancer and other diseases. Nature (London). 2000;407,249-257
Darland DC, D'Amore PA. TGF beta is required for the formation of capillary-like structures in three-dimensional cocultures of 10Tl12 and endothelial cells.Angiogenesis.2001 4(1): 11-20.
Erdag G, Sheridan RL. Fibroblasts improve performance of cultured composite skin substitutes on athymic mice.Burns.2004;30(4):322-8.
Ferrara, N., and Alitalo, K. Clinical applications of angiogenic growth factors and their inhibitors.Nature Med.1999; 5, 1359-1 364.
Gruber R, Kandler B, Holzmann P, Vogele-Kadletz M, Losert U, Fischer MB, Watzek G. Bone marrow stromal cells can provide a local environment that favors migration and formation of tubular structures of endothelial cells. Tissue Eng.2005; 11 (5-6):896-903.
Honorati MC, Neri S, Cattini L, Facchini A. lnterleukin-17, a regulator of angiogenic factor release by synovial fibroblasts. Osteoarthritis artilage.2005;23; [Epub ahead of print].
Hudon V, Berthod F, Black AF, Damour 0, Germain L, Auger FA. A tissueengineered endothelialized dermis to study the modulation of angiogenic and angiostatic molecules on capillary-like tube formation in vitro. Br J Dermatol.2003; 148(6):1094-104.
Jam RK, Au P, Tam J, Duda DG, Fukumura D. Engineering vascularized tissue. Nat Biotechnol.2005;23(7):821 -3.
Keshet, E., and Ben-Sasson, S. A. Anticancer drug targets: approaching angiogenesis. J.CIin. lnvest.1 999;1 04, 1497-1501.
Lazarous DF, Shou M, Stiber JA, Dadhania DM, Thirumurti V, Hodge E, Unger EF. Pharmacodynamics of basic fibroblast growth factor: route of administration determines myocardial and systemic distribution. Cardiovasc Res.1997;36(1):78-85.
Markowicz*, E. Koellensperger, S. Neuss, G.C.M. Steffens and N. PaIlua. Enhancing the Vascularization of Three-Dimensional Scaffolds: New strätegies in Tissue Regeneration and Tissue Engineering. Topics in Tissue Engineering, Volume 2, 2005.
Mertsching H, Walles T, Hofmann M, Schanz J, Knapp WH. Engineering of a vascularized scaffold for artificial tissue and organ generation. Biomaterials. 2005;26(33):661 0-7.
Mooney DJ, Mikos AG. Growing new organs. Sci Am. 1999 Apr;280(4):60-5.
Omaida C. Velazquez, Ruthanne Snyder,Zhao-Jun Liu, Ronald M. Fairman., and Meenhard Herlyn Fibroblast-dependent differentiation of human microvascular endothelial cells into capillary-like, three- dimensional networks. The FASEB Journal express article 10.1096/fj.01-lOllfje. Published online June 7,2002.
Pepper MS, Ferrara N, Orci L, Montesano R. Potent synergism between vascular endothelial growth factor and basic fibroblast growth factor in the induction of angiogenesis in vitro. Biochem Biophys Res Commun.1992;189(2):824-31.
Risau W, Flamme I. Vasculogenesis. Annu Rev Cell Dev Biol. 1995;11:73-91.
Tang YL, Zhao Q, Zhang YC, Cheng L, Liu M, Shi J, Yang YZ, Pan C, Ge J, Phillips Ml. Autologous mesenchymal stem cell transplantation induce VEGF and neovascularization in ischemic myocardium. Regul Pept2004;117:3-10.
Thompson H. G., Truong D. T., Griffith C. K., George S. C. A three-dimensional in vitro model of angiogensis in the airway mucosa. Pulm Pharmacol Ther. 2006, Jan 13.
Velazquez O. C., Snyder R., Lin Z., Fairman R. M., Herlyn M. Fibroblast-dependent differentiation of human microvascular endothelial cells into capillary-like, three-dimensional networks. The FASEB Journal 10.1096;06.2002.
Watanabe M, Fujioka-Kaneko Y, Kobayashi H, Kiniwa M, Kuwano M, Basaki Y. Involvement of integrin-linked kinase in capillary/tube-like network formation of human vascular endothelial cells.Biol Proced Online.2005;7:41-7. Epub 2005 Apr 27.
Yancopoulos, G. D., Davis, S., Gale, N. W., Rudge, J. S., Wiegand, S. J., Holash, J. Vascular-specific growth factors and blood vessel formation. Nature (London).2000;407,242-248.

### Erläuterungen zu den Figuren 1 bis 3

### Figur 1:

VEGF-Quantifizierung in Medien, konditioniert mit kultivierten humanen Blasenstromazellen (b), humanen Knochenmarksstroma-Progenitorzellen (c), humanen Urothelzellen (d) und humanen Urothel-Blasenstromazellen (e), im Vergleich zu nicht-konditionierten Medien (a), wie durch ELISA-Analyse bewertet. Die Medien wurden 72 Stunden, nachdem die Zellen Konfluenz erreichten, geerntet. Jede Säule zeigte den Mittelwert ± Standardabweichung.

### Figur 2:

Relative Zellpopulation von kultivierten Blasen- (A) und dermalen (B) mikrovaskulären Entothelzellen auf azellulärer Matrix, beschickt mit unterschiedlichen Kulturmedien: a- nicht-konditioniertes Medium, b- Medium, konditioniert mit Blasenstromazellen, c- Medium, konditioniert mit Knochenmarksstroma-Progenitorzellen, d- Medium, konditioniert mit Urothelzellen, e- Medium, konditioniert mit Urothel-Blasenstromazellen. (Mittelwerte ± Standardabweichung)

### Figur 3:

Mittelwerte der lichtmikroskopischen Analyse der gesamten Netzwerklängen der gebildeten Röhrchen durch Harnblasen- (A) und dermale (b) mikrovaskuläre Endothelzellen, kultiviert auf azellulären Matrices am Tag 28 nach dem Säen. Die gesamte Netzwerklänge wurde durch mikroskopisches Sichtbarmachen von drei histologischen Längsschnitten, aus drei verschiedenen Konstrukten der Zellen, gewonnen aus jeweils einer Harnblase, bestimmt. Die Länge der Röhrchen wurde durch manuelle Messung berechnet. Mikrovaskuläre Endothelzellkulturen wurden beschickt mit: a- nicht-konditioniertem Medium, b- Medium, konditioniert mit Blasenstromazellen, c- Medium, konditioniert mit Knochenmarksstroma-Progenitorzellen, d- Medium, konditioniert mit Urothelzellen, e- Medium, konditioniert mit Urothel-Blasenstromazellen. (Mittelwerte ± Standardabweichung)

## Patentansprüche

1. Verfahren zur Herstellung eines Gewebetransplantatkonstruktes zur Rekonstruktion eines menschlichen oder tierischen Organs, wobei das Gewebetransplantatkonstrukt aus einer Membran und mikrovaskulären Endothelzellen besteht, umfassend die Schritte
(a) Isolieren von dermalen mikrovaskulären Endothelzellen oder mikrovaskulären Blasen-Endothelzellen;
(b) Aufbringen der mikrovaskulären Endothelzellen auf eine biologisch kompatible azelluläre Membran;
(c) Kultivieren der mikrovaskulären Endothelzellen, die auf die biologisch kompatible azelluläre Membran aufgebracht worden sind, unter stromaler Induktion oder unter epithelial-stromaler Induktion, unter Ausbildung mikrovaskulärer Strukturen, bestehend aus mikrovaskulären Endothelzellen, in der Membran.

2. Verfahren nach Anspruch 1, **dadurch gekennzeichnet, daß** das menschliche oder tierische Organ aus der Gruppe ausgewählt ist, die die Harnblase, den Harnleiter und die Harnröhre umfaßt.

3. Verfahren nach Anspruch 1 oder Anspruch 2, **dadurch gekennzeichnet, daß** die mikrovaskulären Endothelzellen organ-spezifische mikrovaskuläre Endothelzellen sind.

4. Verfahren nach einem der Ansprüche 1 bis 3, **dadurch gekennzeichnet, daß** die mikrovaskulären Endothelzellen autologe mikrovaskuläre Endothelzellen sind.

5. Verfahren nach einem der Ansprüche 1 bis 4, **dadurch gekennzeichnet, daß** die stromale Induktion unter Verwendung von humanen oder tierischen Blasenstromazellen oder von humanen oder tierischen Knochenmarksstroma-Progenitorzellen durchgeführt wird.

6. Verfahren nach einem der Ansprüche 1 bis 5, **dadurch gekennzeichnet, daß** nach Abschluß des Kultivierens der mikrovaskulären Endothelzellen, die auf die biologisch kompatible azelluläre Membran aufgebracht worden sind, unter stromaler Induktion oder unter urothelial-stromaler Induktion (Schritt (c)) weitere, gewebespezifische zellen auf die Membran aufgebracht und dort kultiviert werden.

7. Gewebetransplantatkonstrukt zur Rekonstruktion eines menschlichen oder tierischen Organs, bestehend aus
(a) einer biologisch kompatiblen azellulären Membran; und
(b) mikrovaskulären Endothelzellen, die die Membran durchdringen, wobei die mikrovaskulären Endothelzellen dermale mikrovaskuläre Endothelzellen oder mikrovaskuläre Blasen-Endothelzellen sind;
wobei innerhalb der Membran mikrovaskuläre Strukturen aus den mikrovaskulären Endothelzellen ausgebildet sind.

8. Gewebetransplantatkonstrukt nach Anspruch 7, **dadurch gekennzeichnet, daß** das menschliche oder tierische Organ aus der Gruppe ausgewählt ist, die die Harnblase, den Harnleiter und die Harnröhre umfaßt.

9. Gewebetransplantatkonstrukt nach Anspruch 7 oder Anspruch 8, **dadurch gekennzeichnet, daß** die mikrovaskulären Endothelzellen organ-spezifische mikrovaskuläre Endothelzellen sind.

10. Gewebetransplantatkonstrukt nach einem der Ansprüche 7 bis 9, **dadurch gekennzeichnet, daß** die mikrovaskulären Endothelzellen autologe mikrovaskuläre Endothelzellen sind.

11. Gewebetransplantatkonstrukt nach einem der Ansprüche 7 bis 10, **dadurch gekennzeichnet, daß** die mikrovaskulären Strukturen Lumina umfassen.

12. Gewebetransplantatkonstrukt nach einem der Ansprüche 7 bis 11, **dadurch gekennzeichnet, daß** die mikrovaskulären Strukturen vernetzt sind.

13. Gewebetransplantatkonstrukt nach einem der Ansprüche 7 bis 12, **dadurch gekennzeichnet, daß** die mikrovaskulären Strukturen in vitro ausgebildet worden sind.

## Claims

1. A method for the preparation of a tissue transplant construct for the reconstruction of a human or animal organ, wherein the tissue transplant construct consists of a membrane and microvascular endothelial cells, comprising the steps of
(a) isolating dermal microvascular endothelial cells or microvascular bladder endothelial cells;
(b) applying the microvascular endothelial cells onto a biocompatible acellular membrane;
(c) cultivating the microvascular endothelial cells, which have been applied onto the biocompatible acellular membrane under stromal induction or under epithelialstromal induction to form microvascular structures consisting of microvascular endothelial cells in the membrane.

2. The method according to claim 1, **characterized in that** the human or animal organ is selected from the group comprising the urinary bladder, the ureter, and the urethra.

3. The method according to claim 1 or claim 2, **characterized in that** the microvascular endothelial cells are organ-specific microvascular endothelial cells.

4. The method according to any one of claims 1 to 3, **characterized in that** the microvascular endothelial cells are autologous microvascular endothelial cells.

5. The method according to any one of claims 1 to 4, **characterized in that** the stromal induction is performed by using human or animal bladder stromal cells or human or animal marrow stromal progenitor cells.

6. The method according to any one of claims 1 to 5, **characterized in that** after completion of culturing the microvascular endothelial cells which have been applied onto the biocompatible acellular membrane under stromal induction or under urothelial-stromal induction (step (c)) further tissue-specific cells are applied onto the membrane and cultured thereon.

7. A tissue transplant construct for the reconstruction of a human or animal organ, consisting of
(a) a biocompatible acellular membrane; and
(b) microvascular endothelial cells which penetrate the membrane, wherein the microvascular endothelial cells are dermal microvascular endothelial cells or microvascular bladder endothelial cells,
wherein microvascular structures of the microvascular endothelial cells are formed within the membrane.

8. The tissue transplant construct according to claim 7, **characterized in that** the human or animal organ is selected from the group comprising the urinary bladder, the ureter, and the urethra.

9. The tissue transplant construct according to claim 7 or claim 8, **characterized in that** the microvascular endothelial cells are organ-specific microvascular endothelial cells.

10. The tissue transplant construct according to any one of claims 7 to 9, **characterized in that** the microvascular endothelial cells are autologous microvascular endothelial cells.

11. The tissue transplant construct according to any one of claims 7 to 10, **characterized in that** the microvascular structures comprise lumina.

12. The tissue transplant construct according to any one of claims 7 to 11, **characterized in that** the microvascular structures are cross-linked.

13. The tissue transplant construct according to any one of claims 7 to 12, **characterized in that** the microvascular structures have been developed in vitro.

## Revendications

1. Procédé de fabrication d'une construction de transplantation tissulaire destinée à la reconstruction d'un organe humain ou animal, la construction de transplantation tissulaire se composant d'une membrane et de cellules endothéliales microvasculaires, comprenant les opérations
(a) isolation de cellules endothéliales microvasculaires dermiques ou de cellules endothéliales de vessie microvasculaires ;
(b) application de cellules endothéliales microvasculaires sur une membrane acellulaire compatible biologiquement ;
(c) culture des cellules endothéliales microvasculaires qui ont été appliquées sur la membrane acellulaire compatible biologiquement sous induction du stroma ou sous induction épithéliales du stroma, formant des structures microvasculaires, composées de cellules endothéliales microvasculaires, dans la membrane.

2. Procédé selon la revendication 1 **caractérisé en ce que** l'organe humain ou animal est choisi à partir du groupe qui comprend la vessie, l'uretère et l'urètre.

3. Procédé selon la revendication 1 ou la revendication 2 **caractérisé en ce que** les cellules endothéliales microvasculaires sont des cellules endothéliales microvasculaires spécifiques à l'organe.

4. Procédé selon l'une des revendications 1 à 3 **caractérisé en ce** les cellules endothéliales microvasculaires sont des cellules endothéliales microvasculaires autologues.

5. Procédé selon l'une des revendications 1 à 4 **caractérisé en ce que** l'induction du stroma est exécutée par utilisation de cellules stromales de vessie humaines ou animales ou par cellules progénitrices mésenchymateuses de moelle osseuse humaines ou animales.

6. Procédé selon l'une des revendications 1 à 5 **caractérisé en ce que** à la fin de la culture des cellules endothéliales microvasculaires qui ont été appliquées sur la membrane acellulaire compatible biologiquement, d'autres cellules spécifique au tissu sont appliquées sur la membrane par induction du stroma ou induction urothéliale du stroma (opération (c)) et y sont cultivées.

7. Construction de transplantation tissulaire destinée à la reconstruction d'un organe humain ou animal composée
(a) d'une membrane acellulaire compatible biologiquement ; et
(b) de cellules endothéliales microvasculaires traversant la membrane, les cellules endothéliales microvasculaires étant des cellules endothéliales microvasculaires dermique ou de cellules endothéliales de vessie microvasculaires ;
des structures microvascualire à partir de cellules endothéliales microvasculaires étant formées dans la membrane.

8. Construction de transplantation tissulaire selon la revendication 7 **caractérisée en ce que** l'organe humain ou animal est choisi à partir d'un groupe qui comprend la vessie, l'uretère et l'urètre.

9. Construction de transplantation tissulaire selon la revendication 7 ou la revendication 8 **caractérisée en ce que** les cellules endothéliales microvasculaires sont des cellules endothéliales microvasculaires spécifiques à l'organe.

10. Construction de transplantation tissulaire selon l'une des revendications 7 à 9 **caractérisée en ce que** les cellules endothéliales microvasculaires sont des cellules endothéliales microvasculaires autologues.

11. Construction de transplantation tissulaire selon l'une des revendications 7 à 10 **caractérisée en ce que** les structures microvasculaires comprennent des passages.

12. Construction de transplantation tissulaire selon l'une des revendications 7 à 11 **caractérisée en ce que** les structures microvasculaires sont reliées entre elles.

13. Construction de transplantation tissulaire selon l'une des revendications 7 à 11 **caractérisée en ce que** les structures microvasculaires ont été formées in vitro.
